(19) Europäisches Patentamt
European Patent Office
Office européen des brevets

(11) Publication number: **0 194 807**
**B1**

(12) # EUROPEAN PATENT SPECIFICATION

(45) Date of publication of patent specification: **10.10.90**

(51) Int. Cl.$^5$: **A 61 K 47/00**, A 61 L 25/00, A 61 L 15/44, A 61 K 7/48, C 08 H 1/00

(21) Application number: **86301575.6**

(22) Date of filing: **06.03.86**

(54) **Hydrated adhesive gel and method for preparing the same.**

(30) Priority: **09.03.85 JP 45765/85**
**08.06.85 JP 124740/85**

(43) Date of publication of application:
**17.09.86 Bulletin 86/38**

(45) Publication of the grant of the patent:
**10.10.90 Bulletin 90/41**

(84) Designated Contracting States:
**CH DE FR GB LI NL SE**

(56) References cited:
**FR-A-2 551 660**

**CHEMICAL ABSTRACTS, vol. 91, no. 1, 2nd July 1979, page 287, abstract no. 16228v, Columbus, Ohio, US; P.M. ABDELLA et al.: "A new cleavable reagent for cross-linking and reversible immobilization of proteins"**

(73) Proprietor: **NIPPON OIL AND FATS COMPANY, LIMITED**
**10-1, Yuraku-cho 1-chome**
**Chiyoda-ku Tokyo (JP)**

(72) Inventor: **Doi, Hiroshi**
**13-9 Hoshoen**
**Takarazuka City Hyogo Pref. (JP)**
Inventor: **Inoue, Masanori**
**6-23 Mondo Okada-Cho**
**Nishinomiya City Hyogo Pref. (JP)**

(74) Representative: **Sheader, Brian N. et al**
**Eric Potter & Clarkson St. Mary's Court St. Mary's Gate**
**Nottingham NG1 1LE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 194 807 B1**

Courier Press, Leamington Spa, England.

**Description**

This invention relates to hydrated adhesive gel, especially hydrated adhesive gels for autohesion cataplasma and pack agents having sheet shape.

Hitherto, in order to remove inflammation of muscles caused by a bruise, a sprain, etc., swelling, fever, etc., and alleviate a pain of muscles, etc., the affected part can be treated by a cold or hot compress.

In this case, stable moisture retention and viscoelasticity of the using cataplasma are required without decreasing of water content in the hydrated adhesive gel such as ointments and plasters by bodily temperature, losing the adhesiveness for the sake of drying, and the phenomena of droop and surface tackiness due to moisture absorption and softening caused by sweating.

It is required that the hydrated adhesive gel itself has a sufficient adhesion, and a fixed means such as adhesion sheet is not required in order to protect against slipping of the cataplasma caused by bending and stretching of an applied part of the cataplasma.

Such an autohesion cataplasma is disclosed, for example, in the Japanese Patent Laid-open No. 58—21,613, and obtained by blending acrylic ester copolymer emulsion with a base containing polyvinyl pyrrolidone which is crosslinked by methylvinyl ether/maleic anhydride copolymer to provide autohesion.

Further, according to the invention disclosed in the Japanese Patent Laid-open No. 59—13,718, a compress agent having good adhesibility was obtained by adding dialdehyde starch into an aqueous acid solution of gelatin and polyacrylic acid, and adding a metallic salt or a metallic oxide thereto.

Moreover, in order to give an effect of beauty treatment by removing dirt or keratin, and osmosing beauty ingredients such as vitamin or hormone, etc. into the skin, o/w type emulsion of aqueous high-molecular compound such as polyvinyl alcohol or gelatinous film forming ingredients are commercially available as the pack agents. These agents are able to give an effect of beauty treatment by taking out a necessary amount from a vessel such as a tube, etc. prior to use, applying it to the face skin, etc. to form a film, and peeling off the film or washing the face after drying.

These pack agents require a long time to dry and need to rapidly peel when a visitor suddenly comes in order to avoid the visitor seeing the strange face. It is difficult to apply uniform the hydrated adhesive gel such as ointments or plasters on the skin, when the dry film is stripped off, it tends to be torn. There are disadvantages such as that ingredients for the beauty skin cannot be uniformly provided because of the non-uniformity of thickness. In order to improve these disadvantages, a sheet pack agent which is produced by adding a crosslinking agent such as calcium chloride into an aqueous solution of polyacrylic acid to form hydrous sheet gel, and applied on the skin of face, etc. is known by the Japanese Patent Laid-open No. 58—180,408.

However, the said invention of the Japanese Laid-open No. 58—21,613 concerning to the autohesion cataplasma comprises only blending an acrylic ester copolymer emulsion as an adhesive into a base or crosslinked polyvinyl pyrrolidone. As the result, it has disadvantages that the strength of the adhesion is limited because there is no chemical bonding between the base and the adhesive, and the adhesion lowers with time.

Further, the autohesion cataplasma disclosed in the Japanese Patent Laid-open No. 59—13,718 has good early adhesion. However, with the evaporation of the water content, the adhesion lowers, and especially there are disadvantages that the adhesion is little shown in case of reapplying the agents on the skin once the agents are peeled off.

Moreover, as the sheet pack agent, the said method (the Japanese Patent Laid-open No. 58—180,408) uses crosslinking hydrous gel obtained by adding a crosslinking agent into an aqueous solution of polyacrylic acid and/or a polyacrylate. In this case, as the crosslinking agent, metal salts such as calcium chloride, magnesium chloride, etc., compounds having at least 2 epoxy groups in a molecular such as polyethylene glycol diglycidyl ether, glycerine diglycidyl ether, etc., are given.

However, when the said compounds are used as a crosslinking agent, the early adhesion of the obtained cataplasma is good, but the adhesion is remarkably lowered when the sheet is reapplied on the skin after the sheet is peeled off like as the case of the said autohesion cataplasma described in the Japanese Patent Laid-open No. 59—13,718.

When the latter or compounds having at least 2 epoxy groups in a molecular are used, the reaction between these croslinking agent an aqueous solution of polyacrylic acid and/or a polyacrylate is very slow. Therefore, there are some faults, for example, the reaction needs high temperatures, e.g. 90°C, so that the beauty skin ingredients such as vitamins, etc. which are decomposable require to absorb into the sheet hydrous gel in particular after the formation of the gel.

The inventors have tried to solve the above disadvantages, and found that reaction products by adding an aqueous solution of an N-hydroxyimidoester compound into an aqueous solution of gelatin which contains a protein having amino groups at the side groups thereof and a gelling retarder such as calcium chloride, urea, etc., and partially bridging the protein, have very strong adhesion in wet stage.

Further, the inventors found that by substituting a part of water in hydrated adhesive gel such as an ointment or plaster for a hydrophilic tackifier such as glycerol, ethylene glycol, or polypropylene glycol which is liquid at ordinary temperature, it is possible to protect from reducing adhesibility of said hydrated adhesive gel even if water content decreased during the use of an aqueous adhesive gel.

According to one aspect of the invention there is provided a hydrated adhesive gel using a product

which is obtained by reacting an aqueous solution essentially containing protein selected from gelatin, proteose, pepton, casein, albumin, globulin, prolamin, protamine, histone and glutelin, together with a N-hydroxyimidoester compound represented by the following formula (1).

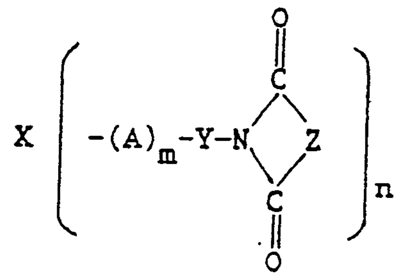

.... (1)

wherein X is a residue of a compound having 2 to 6 carbons and 2 to 6 hydroxyl groups, A is one or more groups selected from oxyethylene, oxypropylene, and oxybutylene groups,

Y is a residue of dibasic acid selected from oxalic acid, malonic acid, succinic acid, glutaric acid, adipic acid, azelaic acid, sebacic acid, decane-dicarboxylic acid, iso succinic acid, methyl succinic acid, malic acid, maleic acid, fumaric acid, tartaric acid, acetondicarboxylic acid, itaconic acid, phthalic acid, isophthalic acid, terephthalic acid, homophthalic acid, hexahydrophthalic acid, tetrahydrophthalic acid, dihydrophthalic acid, p-phenylenediacetic acid, naphthalene-2,3-dicarboxylic acid, diphenic acid, aspartic acid or glutamic acid.

Z is

① a group of (2) 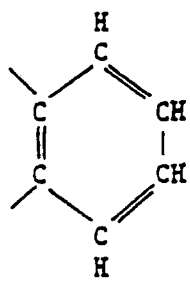 and including compounds of this structure which have been partially substituted

② a group of (3) 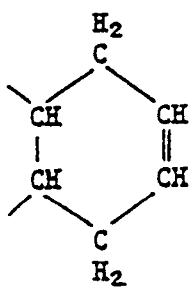 and including compounds of this structure which have been partially substituted

③ a group of (4) 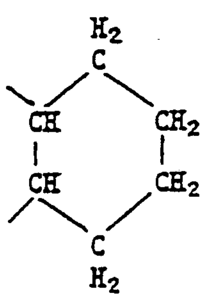 and including compounds of this structure which have been partially substituted

④ a group of (5) 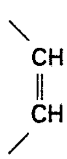 and including compounds of this structure which have been partially substituted

⑤ a group of (6) 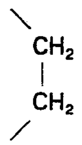 and including compounds of this structure which have been partially substituted

⑥ a group of (7) 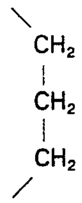 and including compounds of this structure which have been partially substituted

⑦ a group of (8) 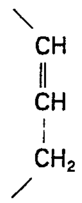 and including compounds of this structure which have been partially substituted

m is 1—3000

n is 2—6

According to another aspect of the invention there is provided a hydrated adhesive gel using a product which is obtained by reacting an aqueous solution essentially containing protein selected from gelatin, proteose, pepton, casein, albumin, globulin, prolamin, protamine, histone and glutelin, a gelling retarder, and a hydrophilic tackifier together with a N-hydroxyimidoester compound represented by the following formula (1).

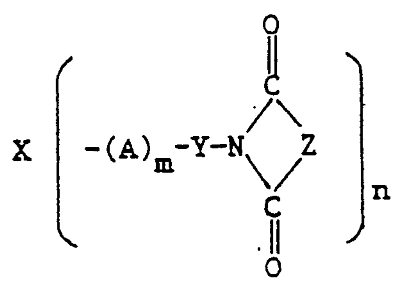 .... (1)

wherein X is a residue of a compound having 2 to 6 carbons and 2 to 6 hydroxyl groups, A is one or more groups selected from oxyethylene, oxypropylene, and oxybutylene groups,

Y is a residue of dibasic acid selected from oxalic acid, malonic acid, succinic acid, glutaric acid, adipic acid, azelaic acid, sebacic acid, decane-dicarboxylic acid, iso succinic acid, methyl succinic acid, malic acid, maleic acid, fumaric acid, tartaric acid, acetondicarboxylic acid, itaconic acid, phthalic acid, isophthalic acid, terephthalic acid, homophthalic acid, hexahydrophthalic acid, tetrahydrophthalic acid, dihydrophthalic acid, p-phenylenediacetic acid, naphthalene-2,3-dicarboxylic acid, diphenic acid, aspartic acid or glutamic acid.

① a group of (2) 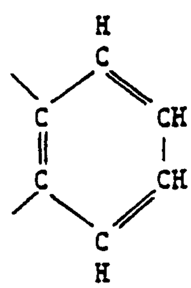 and including compounds of this structure which have been partially substituted

② a group of (3)

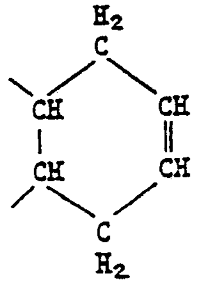

and including compounds of this structure which have been partially substituted

③ a group of (4)

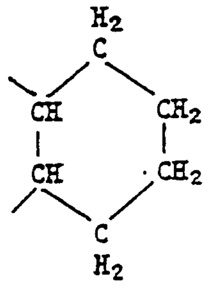

and including compounds of this structure which have been partially substituted

④ a group of (5)

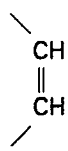

and including compounds of this structure which have been partially substituted

⑤ a group of (6)

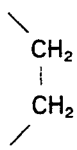

and including compounds of this structure which have been partially substituted

⑥ a group of (7)

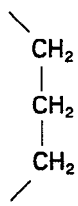

and including compounds of this structure which have been partially substituted

⑦ a group of (8)

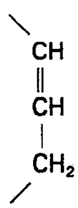

and including compounds of this structure which have been partially substituted

m is 1—3000

n is 2—6

N-hydroxyimidoester compounds (abbreviated as compounds of general formula (1) )represented by the following formula (1) used in this invention are as follows.

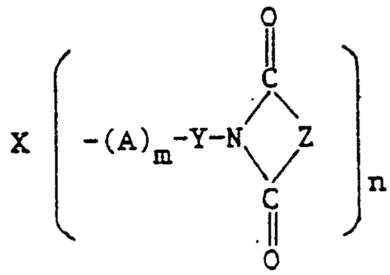 .... (1)

wherein X is an alcohol residue produced from a compound having 2 to 6 carbons and 2 to 6 hydroxyl groups such as ethylene glycol, propylene glycol, glycerol, diglycerol, trimethylolethane, trimethylolpropane, erythritol, pentaerythritol, sorbitol, mannitol, glucose, mannose, xylose, sorbitin, etc., n is a value of 2—6 corresponding to the number of hydroxyl groups.

A is a polymerization unit or a copolymerization unit selected from one kind or any combination of 2 to 3 kinds of hydroxyethylene, hydroxypropylene, and hydroxybutylene groups, m is an average additional mole number thereof.

Examples of the substituents of Z are as follows:
in the case of formula (2),

⑧ 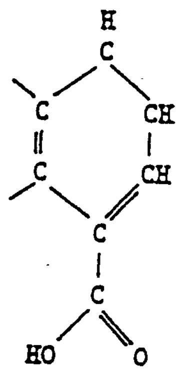 ⑨ 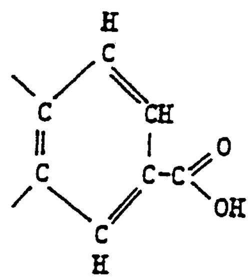

in the case of formula (5),

⑩ 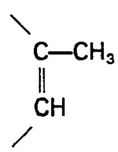 ⑪ 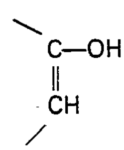

in the case of formula (6),

⑫ 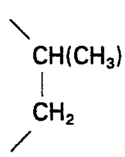 ⑬ 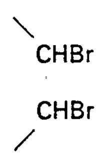

⑭ 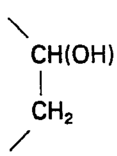 ⑮ 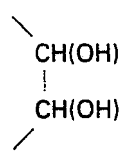

in the case of formula (7),

⑯ 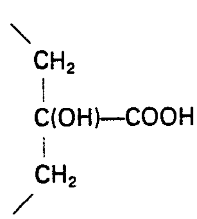 ⑰ 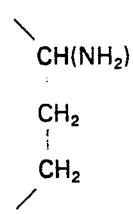

The compounds of the general formula of (1) consisting of the said each constitution react specifically with amino groups, and produce addition products of amino groups with the release of imidoxyl groups, so that the compounds act as crosslinking agents to the protein having amino groups at the side chains of gelatin, etc., as described below, and reacted by crosslinking reaction at ordinary temperatures in an aqueous solution to form good gels by transforming the proteins into macromolecular substances.

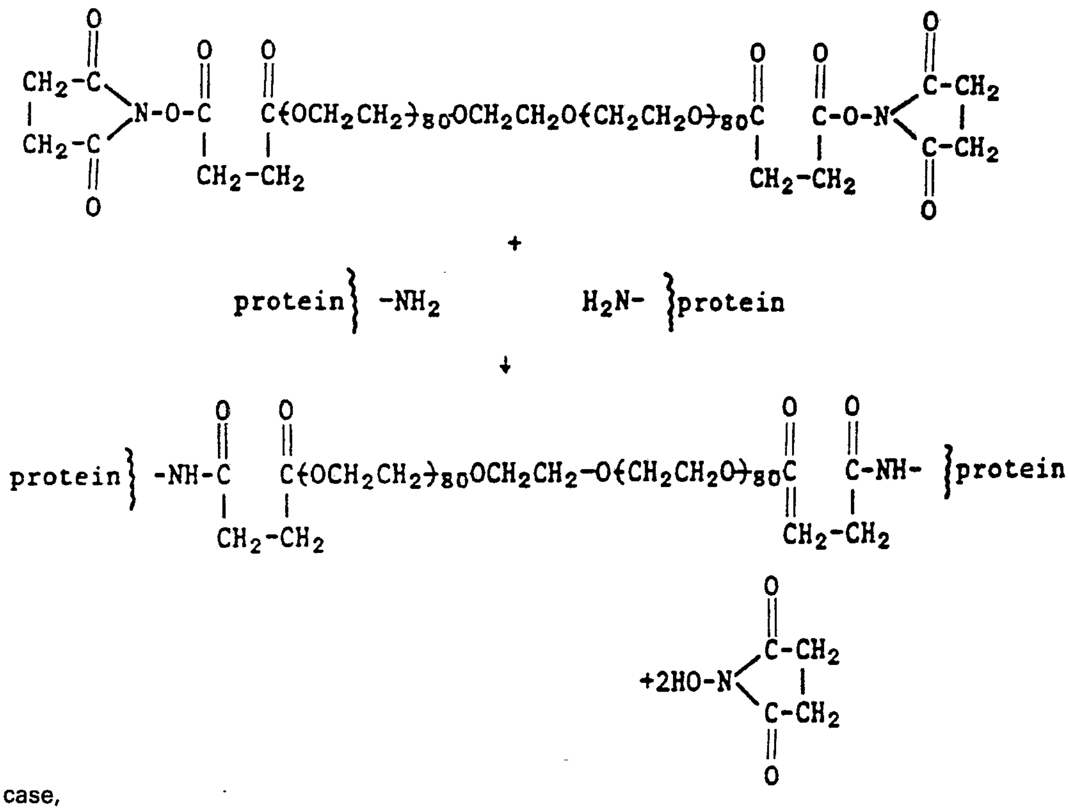

In this case,

when X is an alcohol residue produced from diol such as ethylene glycol, propylene glycol, etc., n is 2, the compound of general formula (1) becomes a bifunctional crosslinking agent, and relatively soft gel such as ointment is produced. When X is an alcohol residue produced from polyol such as pentaerythritol, sorbitol, etc., n is 4 or 6, the compound of general formula (1) becomes polyfunctional crosslinking agent. In producing the gel by this compound, rigid gel having high crosslinking density is produced. When n is greater than 7, obtained gel does not show adhesion. From the said reason, preferable range is 2—3.

Further when A is an oxyethylene group, the hydrophilic nature of the compound of general formula (1) is larger than that of an oxypropylene group. When A is a copolymer of oxyethylene and oxypropylene groups, the hydrophilic nature is varied by the ratio of both, so that it is able to control the degree of the hydrophilic nature.

m is able to take a value ranging 1—3000, when m is lesser, the crosslinking density of the compound per unit weight of general formula (1) becomes higher. Therefore, a rigid gel can be obtained easily, and the hydrophilic nature of the compound of general formula (1) becomes too small. When m is over 3000, the imidoester part of the compound of general formula (1) becomes too small, the compound is impractical because the function as the crosslinking agent is very small. Accordingly, the preferable range of m is 5—200.

However, the dibasic acid in which Y is a residue is optionally selected because the esterification easily occurs between oxyalkylene addition product of alcohol and acid imide.

As the acid imide, phthalimide that partial structure of Z is represented by general formula (2) and the partial substituent thereof, maleimide of general formula (5) and the partial substituent thereof, and succinimide of general formula (6) and the partial substituent thereof are desirable because these compounds are easily produced industrially and cheap.

The gelling retarder used in this invention is a compound which retard the velocity for gradually varying aqueous solution of protein having amino groups at the side chains, such as gelatin dissolved by heating into the gel together with lowering of temperature, and has an effect which allows to lower the gelling temperature. The gelling retarder which is stable as an aqueous solution includes, for example, inorganic compounds containing chlorine such as, potassium chloride, sodium chloride, calcium chloride, magnesium chloride, ammonium magnesium chloride, ammonium chloride, zinc chloride, ammonium zinc chloride, manganese chloride, barium chloride, nickel chloride, lithium chloride, cobalt chloride, aluminum chlorid, antimony pentachloride, stannic chloride, stannous chloride, titanous chloride, titanic

chloride, ferric chloride, ferrous chloride, cupric chloride, etc., inorganic compounds containing bromine such as potassium bromide, sodium bromide, calcium bromide, magnesium bromide, ammonium bromide, zinc chloride, manganese bromide, barium bromide, nickel chloride, lithium bromide, aluminum bromide, stannous bromide, ferrous bromide, ferric bromide, cupric bromide etc., inorganic compounds containing a nitrate group such as potassium nitrate, sodium nitrate, calcium nitrate, ammonium nitrate, zinc nitrate, barium nitrate, nickel nitrate, aluminum nitrate, cobalt nitrate, magnesium nitrate, manganese nitrate, lithium nitrate, ferrous nitrate, ferric nitrate, silver nitrate, cupric nitrate, etc., inorganic compounds containing a thiocyanate group such as potassium thiocyanate, sodium thiocyanate, calcium thiocyanate, ammonium thiocyanate, barium thiocyanate, ferric thiocyanate, etc., nonelelctrolytes such as resorcinol, hydroquinone, pyrocatechol, pyrogallol, furfural, urea, ethylalcohol, ethylalcohol denatured with methylalcohol, isopropylalcohol, chlorobutylalcohol, erythritol, etc.

The compounding weight of the gelling retarder 0.05—5 times of the compounding weight of the protein having amino groups, preferably 0.5—1.5 times.

If the hydrophilic tackifier used in this invention is dissolved in water and the water evaporates during the use of the hydrated adhesive gel, such as ointment or plaster, it remains in said hydrated adhesive gel, such as the ointment or plaster and gives the adhesion properties to the gel. The hydrophilic tackifier includes glycerol, ethylene glycol, propylene glycol, and polyethylene glycol and polypropylene glycol which are liquid at ordinary temperatures, etc. These tackifiers have a tackifier effect by themselves. However, the effect is also represented by the combined use.

The blending weight of the hydrophilic tackifier used in the hydrated adhesive gel is 1—80 weight% preferably 3—50 weight%.

When the hydrated adhesive gel of this invention uses as a medicine for external use, efficacious ingredients such as methyl salicylate, glycol salicylate, menthol, camphol, thymol, borneol, diphenhydramine, indomethacin, ketoprophen, bruphen, nitroglycerol, peppermint oil, hormones, vitamins, etc., humectants such as sorbitol, benzyl alcohol, etc., powder base such as kaolin, bentonite, zinc white, titanium, dioxide, etc., as desired, tackifier such as rosin, ester-gum, polybutene, etc., cationic, anionic and nonionic surface active agents, other water-soluble or hydrophilic synthetic high molecular compounds or natural high molecular compounds, etc. such as polyvinyl alcohol, carboxymethylcellulose, arabic gum, polyvinylpyrrolidone, polyacrylic acid, pectin, etc.

Further, when the hydrated adhesive gel such as ointment or plaster of this invention is used as a cosmetic pack agent having sheet structure, it is possible to add nutritive agents of the skin such as vitamins, hormones, amino acids, materials extracted or secreted from animal or plant tissues, etc., ingredients for beautiful skin used in usual pack agents, for example, skin improvement agents, etc., such as bleaching agents, depilatory agents, etc., coloring agents such as titanium dioxide, red 2 for food, as desired, hydrophilic synthetic high molecular compounds or natural high molecular compounds, etc., such as polyacrylic acid polyvinyl pyrrolidone, pectin, etc.

The hydrated adhesive gel of this invention is an aqueous gel in which the base contains crosslinking protein. If once such hydrated adhesive gel is adhered on the skin, the adhesion on the skin is very strong, so that such gel can be reapplied after peeling off, as desired. Its tack reduction is rarely found. Further, the early tackiness does not lower with time, and the tackiness tends to increase with the evaporation of water content. It is possible to raise the water retention rate in said gel, and it is possible to use up to 70 weight% according to compositions. When the hydrated adhesive gel of this invention having such good properties is used as the cataplasma, the said gel has good shape retention, moisture retention, adhesion to the skin, maintenance of cold feeling. Especially, the agents have good adhesion to the skin. There is no deviation or release of applied parts by bodily exercise. Moreover, when said adhesive gel is reapplied after releasing, the adhesion is good. The lowering of adhesion by sweating is very little. Even if the ointment is used for a long time releasing of the cataplasma is very difficult because the adhesion is not lowered by the evaporation of water content. When the cataplasma is released after using, there is not a pain in comparison with the conventional cataplasma using adhesion sheets. There is no poisoning of the skin compared with using the adhesion sheets.

When the ointment of this invention is used as a sheet pack agent for beauty effects of the skin, especially for the face skin, disadvantages of conventional jellied pack agents, namely, long drying time, impossible reusing of used films, non-uniformed films, etc. are dissolved. In comparison with the sheet pack agents obtained by crosslinking polyacrylate with crosslinking agents such as calcium chloride, etc., in an aqueous solution, the ointment of this invention has good adhesion and a remarkable effect in the adhesion of the reapplied pack agent.

The invention will now be described in detail with reference to the accompanying drawings, wherein:

Fig. 1 shows adhesion change with time of Examples 1—7 and Comparison examples 1 and 2;

Fig. 2 shows repeated adhesion of Examples 1—7 and Comparison examples 1 and 2;

Fig. 3 shows adhesion change with time of Examples 8—10 and Comparison examples 3 and 4; and

Fig. 4 shows repeated adhesion of Examples 8—10 and Comparison examples 3 and 4.

Other objects and advantages of this invention will become apparent with reference to the following Examples.

(Experiments and Comparison examples)

Preparation of the compounds of the general formula (1) (abbreviated as crosslinking agents).

Preparation example 1:

141 mol of additional mol numbers of polyethylene glycol is reacted with 2 mol of maleic anhydride to form a half ester and the ester is reacted with 2 mol of N-hydroxysuccinic imide to prepare a crosslinking agent 1.

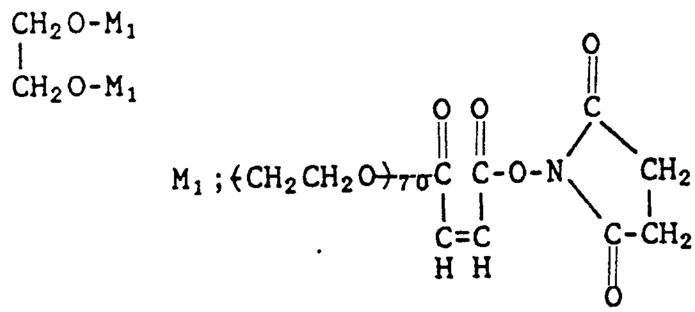

Preparation example 2:

1 mol of glycerol is addition-polymerized with 1050 mol of propylene oxide, the obtained polypropylene glycol ether of glycerol is reacted with 3 mol of phthalic anhydride to form a half ester, and the ester is reacted with 3 mol of N-hydroxyphthalic imide to prepare a crosslinking agent 2.

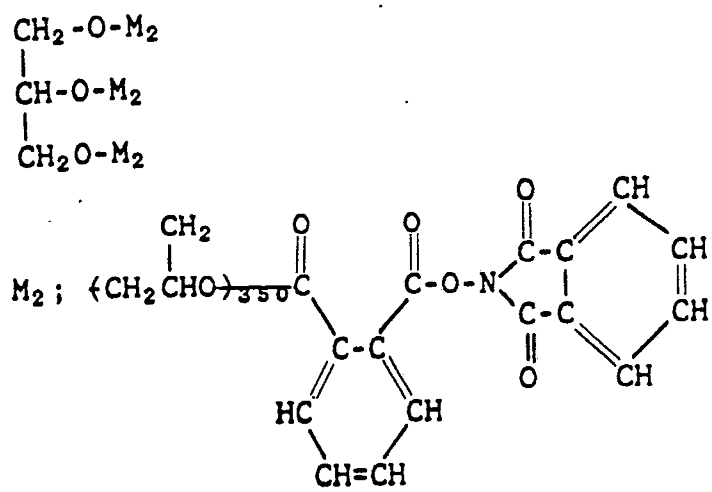

Preparation example 3:

1 mol of pentaerythritol is addition-polymerized with 12000 mol of ethylene oxide, the obtained polyethylene glycol ether of pentaerythritol is reacted with 4 mol of citraconic anhydride to form a half ester, and the ester is reacted with 4 mol of N-hydroxymaleic imide to prepare a crosslinking agent 3.

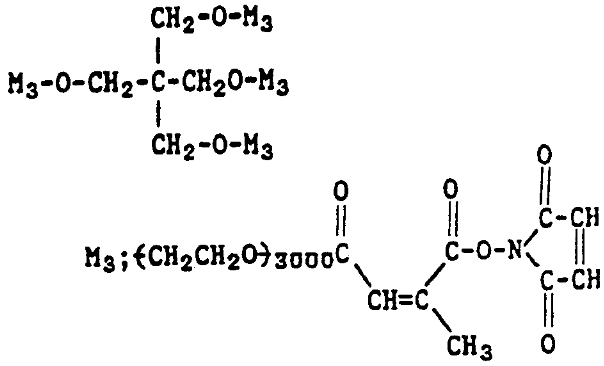

Preparation example 4:

1701 mol of additional mol numbers of polypropylene glycol is reacted with 2 mol of succinic anhydride to form a half ester, and the ester is reacted with 2 mol of N-hydroxyglutaconimide to prepare a crosslinking agent 4.

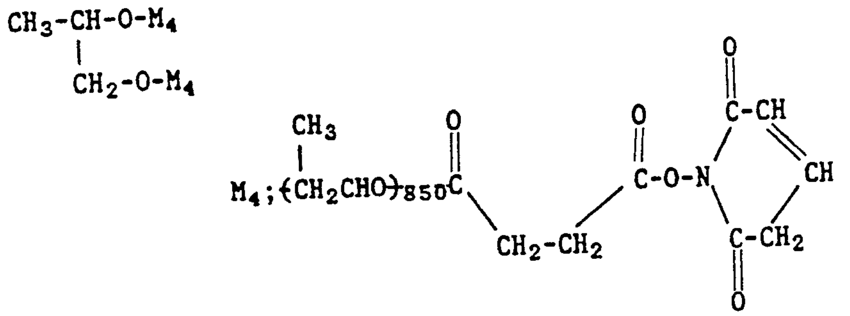

Preparation example 5:

1 mol of glucose is addition-polymerized with 25 mol of ethylene oxide and 25 mol of propylene oxide, the obtained glucose-polyoxyalkylene glycol ether compound is reacted with 5 mol of tartaric anhydride to form a half ester, and the ester is reacted with 5 mol of N-hydroxyglutal imide to prepare a crosslinking agent 5.

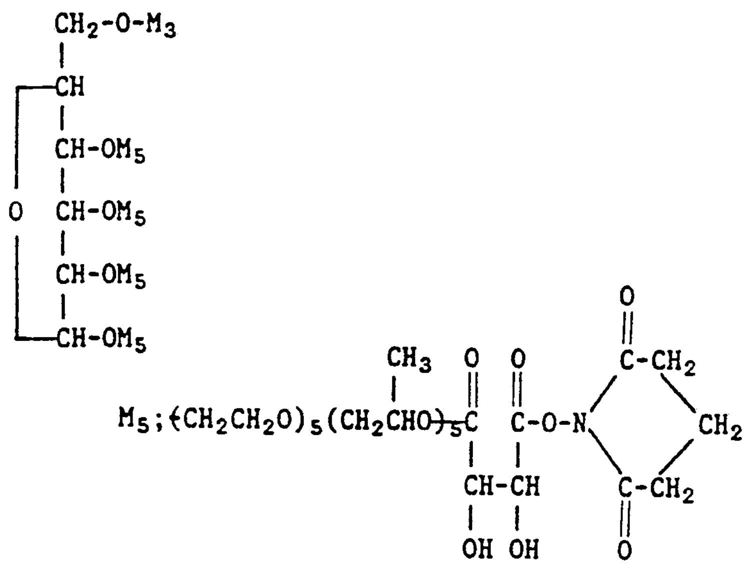

Preparation example 6:

1 mol of glycerol is addition-polymerized with a mixture of 24 mol of ethylene oxide and 6 mol of butylene oxide, the obtained glucose-polyoxyalkylene glycol ether compound is reacted with 3 mol of succinic anhydride to form a half ester, and the ester is reacted with 3 mol of N-hydroxysuccinic imide to prepare a crosslinking agent 6.

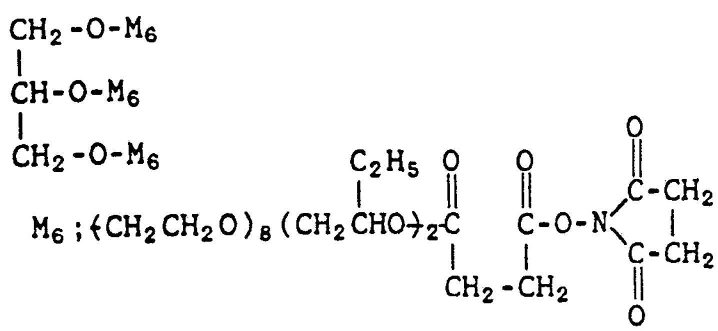

Example 1

Based on the compounding as shown in Table 1, a raw material No. 1 is added into 9/10 of a raw

material No. 36 and heated to dissolve at 60—70°C. Then, a raw material No. 3 is added to the solution,, stirred and dissolved. Further, materials No. 18 and No. 26 are added and stirred with a dissolver to disperse. To the dispersed solution, materials No. 28, 29, 30 and 33 are added, stirred with the dissolver at 2000 rpm for 5 minutes to disperse and obtained $A_1$ liquid. On the other hand, $B_1$ liquid is obtained by adding 1/10 of the material No. 36 into a material No. 12, stirring and dissolving.

After $A_1$ liquid is added to $B_1$ liquid, stirred and mixed, the solution is applied to a piece of non-woven fabric. Then, the cataplasma of Example 1 is obtained by facing with polyethylene film.

Using the cataplasma, the changing of the adhesion with time is measured by the method as shown below. The result is shown in Fig. 1.

(Method for measuring the adhesion)

A cataplasma cut 2 cm squares is applied on a flat part of an arm. After a certain time, an acrylic resin plate of 2 cm squares and 1 mm thickness having a reverse U type puller at the center is applied by an adhesive onto the cataplasma. After 10 minutes, the puller is pulled up to a vertical direction with a spring balance of 500 g having a hook and the adhesion is measured.

The properties of strike-through, shaping retention, adhesion and maintenance of cool-feeling are also tested.

The result is shown in Fig. 1 and Table 2.

(Method for measuring the adhesion of repetition)

According to the method for measuring the first adhesion of the cataplasma is measured. Then, the tested material is applied again on the arm, and, after 10 minutes, the adhesion is measured again. This action is repeated 4 times.

The result is shown in Fig. 2.

Experiment 2

Based on the compounding as shown in Table 1, the raw material No. 1 is added into 9/10 of the raw material No. 36 and heated to dissolve at 60—70°C. Then a raw material No. 4 is added to the solution, stirred and dissolved. Further, materials Nos. 21 and 26 are added and stirred with the dissolver to disperse. To the dispersed solution, materials Nos. 27, 29, 30, 31 and 33 are added, stirred with the dissolver at 2000 rpm for 5 minutes to disperse and obtained $A_2$ liquid. On the other hand, $B_2$ liquid is obtained by adding 1/10 of the material No. 36 into a material No. 13, stirring and dissolving.

After $A_2$ liquid is added to $B_2$ liquid, stirred and mixed, the solution is applied to a piece of non-woven fabric. Then, the cataplasma of Example 2 is obtained by facing with polyethylene film.

Testing with the same cataplasma test method as Example 1, the result is shown in Figs. 1 and 2 and Table 2.

Experiment 3

Based on the compounding as shown in Table 1, the raw material No. 1 is added into 1/2 of the raw material No. 36 and heated to dissolve at 70—80°C. Then a raw material No. 5 is added to the solution, stirred and dissolved. Further, materials Nos. 20, 28, 29, 30 and 33 are added, stirred with the dissolver at 1500 rpm for 10 minutes to disperse and obtained $A_3$ liquid. On the other hand, $B_3$ liquid is obtained by adding 1/2 of the material No. 36 into a material No. 14, stirring and dissolving.

After $A_3$ liquid is added to $B_3$ liquid, stirred and mixed, the solution is applied to a piece of non-woven fabric. Then, the cataplasma of Example 3 is obtained by facing with polypropylene film.

Testing with the same cataplasma test method as Example 1, the result is shown in Figs. 1 and 2 and Table 2.

Example 4

Based on the compounding as shown in Table 1, the raw material No. 1 is added into 3/4 of the raw material No. 36 and heated to dissolve at 90—100°C. Then, a raw material No. 6 is added to the solution, stirred and dissolved. Further, materials Nos. 10, 22, 24 and 26 are added and stirred with the dissolver to disperse. To the dispersed solution, materials Nos. 27, 29, 30 and 33 are added, stirred with the dissolver at 2500 rpm for 10 minutes to disperse and obtained $A_4$ liquid. On the other hand, $B_4$ liquid is obtained by adding 1/4 of the material No. 36 into a material No. 15, stirring and dissolving.

After $A_4$ liquid is added to $B_4$ liquid, stirred and mixed, the solution is applied to a piece of non-woven fabric. Then, the cataplasma of Example 4 is obtained by facing with polypropylene film.

Testing with the same cataplasma test method as Example 1, the result is shown in Figs. 1 and 2 and Table 2.

Example 5

Based on the compounding as shown in Table 1, the raw material No. 1 is added into 9/10 of the raw material No. 36 and heated to dissolve at 90—100°C. Then, a raw material No. 7 is added to the solution, stirred and dissolved. Further, materials Nos. 18, 19, 23 and 25 are added and stirred with a dissolver to disperse. To the dispersed solution, materials Nos. 28, 29, 31 and 33 are added, stirred with the dissolver at

2500 rpm for 10 minutes to disperse and obtained $A_5$ liquid. On the other hand, $B_5$ liquid is obtained by adding 1/4 to 1/10 of the material No. 36 into a material No. 16, stirring and dissolving.

After $A_5$ liquid is added to $B_5$ liquid, stirred and mixed, the solution is applied to a piece of non-woven fabric. Then, the cataplasma of Example 5 is obtained by facing with polypropylene film.

Testing with the same cataplasma test method as Example 1, the result is shown in Figs. 1 and 2 and Table 2.

Experiment 6

Based on the compounding as shown in Table 1, the raw material No. 1 is added into 9/10 of the raw material No. 36 and stirred to dissolve at 25—35°C. Then, raw materials Nos. 18 and 26 are added to the solution, stirred and dissolved. Further, materials Nos. 32 and 33 are added, stirred with the dissolver at 2000 rpm for 15 minutes to disperse and obtained $A_6$ liquid. On the other hand, $B_6$ liquid is obtained by adding 1/10 of the material No. 36 into a material No. 17, stirring and dissolving.

After $A_6$ liquid is added to $B_6$ liquid, stirred and mixed, the solution is applied to a piece of non-woven fabric. Then, the cataplasma of Example 6 is obtained by facing with polypropylene film.

Testing with the same cataplasma test method as Example 1, the result is shown in Figs. 1 and 2 Table 2.

Experiment 7

Based on the compounding as shown in Table 1, the raw material No. 1 is added into 9/10 of the raw material No. 36 and heated to dissolve at 60—70°C. Then, raw material Nos. 18 and 26 are added to the solution, stirred with a dissolver and dispersed. Further, materials Nos. 28, 29, 30 and 33 are added, stirred with the dissolver at 2000 rpm for 5 minutes to disperse and obtained $A_{(1)}$ liquid. On the other hand, $B_{(1)}$ liquid is obtained by adding 1/10 of the material No. 36 into the material No. 12, stirring and dissolving.

After $A_{(1)}$ liquid is added to $B_{(1)}$ liquid, stirred and mixed, the solution is applied to a piece of non-woven fabric. Then, the cataplasma of Example 7 is obtained by facing with polyethylene film.

Testing with the same cataplasma test method as Example 1, the result is shown in Figs. 1 and 2 and Table 2.

Comparative example 1

Based on the compounding as shown in Table 1, the raw material No. 1 is added into 9/10 of the raw material No. 36 and heated to dissolve. Then, raw materials Nos. 8, 10, 18, 23, 27 and 29 are added, stirred with the dissolver at 2500 rpm for 10 minutes to disperse and the obtained ointment or plaster is applied to a piece of non-woven fabric. Then, the cataplasma of Comparative example 1 is obtained by facing with polyethylene film.

Testing with the same cataplasma test method as Example 1, the result is shown in Figs. 1 and 2 and Table 2.

Comparative example 2

Based on the compounding as shown in Table 1, a raw material No. 34 is added into a raw material No. 9, raw materials Nos. 28, 29 and 30 are added and homogeneously stirred, and then a solution of 1/2 of the raw material No. 36 dissolving a material No. 11 is added and homogeneously mixed, then 1/2 of the raw material No. 36 is added, and a raw material No. 35 is added and stirred sufficiently. The obtained ointment or plaster is applied to a piece of non-woven fabric. Then, the cataplasma of Comparative example 2 is obtained by facing with polythylene film.

Testing with the same cataplasma test method as Example 1, the result is shown in Figs. 1 and 2 and Table 2.

As shown in the test result of Fig. 1, Examples 1—7 show high early adhesion, the adhesion increasing along with the evaporation of water content with time, and maintenance of remarkably high adhesion. However, Comparative example 1 shows low early adhesion, and the adhesion is not increased along with the evaporation of water content with time. And the value after 120 minutes is not arrived at the value of the early adhesion of Examples 1—7. In Comparative example 2, the adhesion is in the range of the adhesion of Examples 1—7 until 60 minutes. However, after the time, the adhesion gradually lowers.

Further, from the test result of Fig. 2, Examples 1—7 show repeatable adhesion. After the sample of the cataplasma is applied, it is peeled off and reapplied, and then peeled off. The said action is repeated 4 times. As the result, the adhesion does not lower, and the readhesion shows above 45 $kg/cm^2$ to 45 $g/cm^2$. However, in Comparative example 1, the early adhesion is low and shows 8 $g/cm^2$, and the readhesion is also 10 $g/cm^2$. In Comparative example 2, the early adhesion is 60 $g/cm^2$, the value is considerably high. In spite of this, the first readhesion is 26 $g/cm^2$ and the value is very low. 4th of the readhesion is very low 18 $g/cm^2$

Table 1(a)

(Unit: parts by weight)

| No. | Raw material | Example | | | | | | | Comparative example | |
|---|---|---|---|---|---|---|---|---|---|---|
| | | 1 | 2 | 3 | 4 | 5 | 6 | 7 | 1 | 2 |
| 1 | Gelatin | 12 | 8 | 6 | 10 | 15 | | 15 | 8 | |
| 2 | Albumin (egg albumin) | | | | | | 15 | | | |
| 3 | Calcium chloride | 12 | | | | | | | | |
| 4 | Zinc chloride | | 7 | | | | | | | |
| 5 | Potassium nitrate | | | 7 | | | | | | |
| 6 | Resorcinol | | | | 4 | | | | | |
| 7 | Urea | | | | | 15 | | | | |
| 8 | Polyvinyl alcohol | | | | | | | | 2 | |
| 9 | Polyacrylate aqueous solution (solid content 25%) | | | | | | | | | 30 |
| 10 | Carboxymethyl cellulose | | | | 1 | | | | 1 | |
| 11 | Ethylene glycol glycidyl ether | | | | | | | | | 0.5 |
| 12 | Crosslinking agent 1 | 2.5 | | | | | | 2 | | |
| 13 | Crosslinking agent 2 | | 4 | | | | | | | |

Table 1(b)

| No. | Raw material | Example | | | | | | | Comparative example | |
|---|---|---|---|---|---|---|---|---|---|---|
| | | 1 | 2 | 3 | 4 | 5 | 6 | 7 | 1 | 2 |
| 14 | Crosslinking agent 3 | | | 24 | | | | | | |
| 15 | Crosslinking agent 4 | | | | 8 | | | | | |
| 16 | Crosslinking agent 5 | | | | | 3 | | | | |
| 17 | Crosslinking agent 6 | | | | | | 5 | | | |
| 18 | Glycerol | 22 | | | | 5 | 10 | 24 | 15 | |
| 19 | Ethylene glycol | | | | | 5 | | | | |
| 20 | Propylene glycol | | | 12 | | | | | | |
| 21 | Polyethylene glycol #200 | | 12 | | | | | | | |
| 22 | Polypropylene glycol #400 | | | | 10 | | | | | |
| 23 | Kaolin | | | | | 1 | | | 20 | |
| 24 | Bentonite | | | | 3 | | | | | |
| 25 | Zinc white | | | | | 1 | | | | |
| 26 | Titanium dioxide | 2 | 2 | | 2 | | 2 | 2 | | |

Table 1(c)

| No. | Raw material | Example | | | | | | | Comparative example | |
|---|---|---|---|---|---|---|---|---|---|---|
| | | 1 | 2 | 3 | 4 | 5 | 6 | 7 | 1 | 2 |
| 27 | Methyl salicylate | | 0.8 | | 3 | | | | 2 | |
| 28 | Glycol salicylate | 1.4 | | 1 | | 5 | | 1.2 | | 3 |
| 29 | ℓ-Menthol | 0.7 | 0.4 | 0.5 | 0.5 | 1 | | 0.6 | 1 | 1.5 |
| 30 | dℓ-Camphol | 0.7 | 0.4 | 0.5 | 0.5 | | | 0.6 | | 0.5 |
| 31 | Thymol | | 0.2 | | | 0.5 | | | | |
| 32 | Indomethacin | | | | | | 4 | | | |
| 33 | Nonionic surface active agent | 1 | 0.5 | 0.5 | 1.5 | 2 | 1 | 1 | | |
| 34 | Sodium hydroxide | | | | | | | | | 0.6 |
| 35 | 5% potassium alum aqueous solution | | | | | | | | | 1 |
| 36 | Water | 45.7 | 64.7 | 48.5 | 56.5 | 46.5 | 63 | 53.6 | 51 | 62.9 |
| Total | | 100 | 100 | 100 | 100 | 100 | 100 | 100 | 100 | 100 |

Table 2

| | Strike-through (note 1) | Shaping retention (note 2) | Adhesion (note 3) | Maintenance of cool-feeling (note 4) |
|---|---|---|---|---|
| Example 1 | o | o | o | o |
| Example 2 | Δ | o | o | o |
| Example 3 | o | o | o | o |
| Example 4 | o | o | o | o |
| Example 5 | o | o | o | o |
| Example 6 | o | o | o | o |
| Example 7 | o | o | o | o |
| Comparative Example 1 | o | x | x | x |
| Comparative Example 2 | x | o | Δ | Δ |

Note 1: Strike-through test

The strike-through of cataplasma to back surface of non-woven fabric is evaluated by the following evaluation modes:

o none,

Δ partial and

x remarkable.

Note 2: Shaping retention

With cataplasma, it is evaluated by the following evaluation modes:

o Droop by bodily temperature or sweating is not entirely recognized.

Δ A part softens and droops.

x Droop

Note 3: Adhesion

o Adhesion feeling to the skin is good, the adhesion between the skin and the ointment or plaster is not released by bending and stretching of the applied part.

Δ Adhesion feeling to the skin is good, but sometimes, the adhesion releases with time.

x Adhesion feeling to the skin is weak.

Note 4: Maintenance of cool-feeling

The feeling in case of using the cataplasma for 12 hours is evaluated.

o There is cool-feeling after 12 hours.

Δ There is cool-feeling after 6 hours.

x There is no cool-feeling after 6 hours.

Table 3(a)

(Unit: Parts by weight)

| No. | Raw material | Example | | | Comparative example | |
|---|---|---|---|---|---|---|
| | | 8 | 9 | 10 | 3 | 4 |
| 1 | Gelatin | 12 | | | | |
| 2 | Albumin (egg albumin) | | 20 | | | |
| 3 | Casein (milk casein) | | | 18 | | |
| 4 | Polyacrylate aqueous solution (solid content 25%) | | | | 60 | 72 |
| 5 | Calcium chloride | 12 | | | 8 | |
| 6 | Magnesium chloride | | | 8 | | |
| 7 | Crosslinking agent 1 | 2.5 | | | | |
| 8 | Crosslinking agent 2 | | | 9 | | |
| 9 | Crosslinking agent 6 | | 6 | | | |
| 10 | Triglycidyl cyanulate | | | | | 0.1 |
| 11 | Glycerol | 22 | 20 | | 10 | 7 |
| 12 | Ethylene glycol | | | 12 | | |

Table 3(b)

| No. | Raw material | Example | | | Comparative example | |
|---|---|---|---|---|---|---|
| | | 8 | 9 | 10 | 3 | 4 |
| 13 | Ascorbic acid | 1.5 | | 2 | 2 | |
| 14 | Tocopherol | | 2 | | | |
| 15 | Paraoxy benzoic acid | 0.2 | | | | 0.2 |
| 16 | Titanium dioxide | | | 3 | | |
| 17 | Nonionic surface active agent | | 1 | | | |
| 18 | Sodium hydroxide | | | 2 | 5 | 6 |
| 19 | Water | 49.8 | 51 | 46 | 15 | 14.7 |
| Total | | 100.0 | 100 | 100 | 100 | 100.0 |

As shown in the result of Table 2, Examples 1—7 show that the cataplasma are excellent in strike-through, shaping retention, adhesion and maintenance of cool-feeling, especially more superior in adhesion and maintenance of cool-feeling than that of Comparative examples 1 and 2.

Experiment 8

Based on the compounding as shown in Table 3, the raw material No. 1 is added into 9/10 of a raw material No. 19 and heated to dissolve at 60—70°C. Then, the raw material No. 5 is added to the solution, stirred and dissolved. Further, materials Nos. 11, 13 and 15 are added, stirred with the dissolver at 1000 rpm for 5 minutes to disperse and obtained $A_8$ liquid. On the other hand, $B_8$ liquid is obtained by adding 1/10 of the material No. 19 into the material No. 7, stirring and dissolving.

After $A_8$ liquid is added to $B_8$ liquid, stirred and mixed at room temperature for 10 minutes, the solution is poured into a polypropylene vessel of 200 × 250 × 2 mm at the thickness of 0.6 mm, and warmed at 2 minutes at 50°C. The obtained gel is applied to a piece of non-woven fabric by softly pushing. After cooling to the room temperature, the gel is peeled off from the vessel. A polypropylene sheet is adhered to the opposite side of the gel sheet, and the sheet cataplasma of Example 8 is obtained.

Testing with the same cataplasma test method as Example 1, the measurement of adhesion and repeated adhesion is conducted, and the result is shown in Figs. 3 and 4.

Experiment 9

Based on the compounding as shown in Table 3, a raw material No. 2 is added into 8/10 of the raw material No. 19 and heated to dissolve at room temperature for 10 minutes. Then, materials Nos. 11, 14 and 17 are added, stirred with the dissolver at 1500 rpm for 10 minutes to disperse and obtained $A_9$ liquid. On the other hand, $B_9$ liquid is obtained by adding 2/10 of the material No. 19 into the material No. 6, stirring and dissolving.

After $A_9$ liquid is added to $B_9$ liquid, stirred and mixed at room temperature for 10 minutes, the solution is poured into a polypropylene vessel of 200 × 250 × 2 mm at the thickness of 0.6 mm warmed at 5 minutes at 40°C, and the thus obtained gel is applied to a piece of non-woven fabric of 200 × 250 × 1 mm by softly pushing. After cooling to the room temperature, the gel is peeled off from the vessel. A polypropylene sheet is adhered to the opposite side of the gel sheet, and the sheet pack of Example 9 is obtained.

Testing with the same pack test method as Example 1, the measurement of adhesion and repeated adhesion is conducted, and the result is shown in Figures 3 and 4.

Experiment 10

Based on the compounding as shown in Table 3, the raw material No. 18 is added into 8/10 of the raw material No. 19. Then, the raw material No. 3 is added, and stirred to dissolve at room temperature for 10 minutes. Then, the raw material No. 6 is added to the solution, stirred and dissolved. Further, materials Nos. 12, 13 and 16 are added, stirred with the dissolver at 1500 rpm for 5 minutes to dissolve and disperse and obtained $A_{10}$ liquid. On the other hand, $B_{10}$ liquid is obtained by adding 2/10 of the material No. 19 into the material No. 8, stirring and dissolving.

After $A_{10}$ liquid added to $B_{10}$ liquid, stirred and mixed at room temperature for 5 minutes, the solution is poured into a polypropylene vessel of 200 × 250 × 2 mm at the thickness of 0.6 mm warmed at 1 minute at 60°C, and the obtained gel is applied to a piece of non-woven fabric of 200 × 250 × 1 mm by softly pushing. After cooling to the room temperature, the gel is peeled off from the vessel. A polypropylene sheet is adhered to the opposite side of the gel sheet, and the sheet pack of Example 10 is obtained.

Testing with the same pack test method as Example 1, the measurement of adhesion and repeated adhesion is conducted, and the result is shown in Figs. 3 and 4.

Comparative example 3:

Based on the compounding as shown in Table 3, the raw material No. 18 is added into the raw material No. 4 and heated to dissolve at room temperature for 10 minutes. Then, raw materials Nos. 11 and 13 are added, stirred with the dissolver at 1000 rpm for 5 minutes to dissolve. Then, materials Nos. 5 and 19 are added and stirred homogeneously at room temperature. The obtained gel solution is applied to a side surface of non-woven fabric at 0.6 mm thickness. A polyester film is adhered to the opposite side surface of the fabric, put into a closing bag, and heated at 60°C for 5 minutes, and the sheet pack of Comparative example 3 is obtained.

Testing with the same pack test method as Example 1, the measurement of adhesion and repeated adhesion is conducted, and the result is shown in Figs. 3 and 4.

Comparative example 4:

Based on the compounding as shown in Table 3, the raw material No. 18 is added into the raw material No. 4 and heated to dissolve at room temperature for 10 minutes. Then, raw materials Nos. 11 and 15 are added, stirred with the dissolver at 1500 rpm for 5 minutes to dissolve. Then, materials Nos. 10 and 19 are added and stirred homogeneously at room temperature. The obtained gel solution is applied to a side surface of non-woven fabric at 0.6 mm thickness. A polyester film is adhered to the opposite side surface of the fabric, put into a closing bag, and heated at 60°C for 5 minutes, and the sheet pack of Comparative

example 4 is obtained.

Testing with the same test method as Example 1, the measurement of adhesion and repeated adhesion is conducted, and the result is shown in Figs. 3 and 4.

As shown in the result of Fig. 3, Examples 8—10 show high early adhesion, the adhesion increasing along with the evaporation of water content with time, and maintenance of remarkably high adhesion. However, in Comparative example 3, the early adhesion is in the range of the value of Examples 8—10, and after that, the adhesion lowers. In Comparative example 4, the early adhesion is low, and the increase of the adhesion with time is also little.

By the high adhesion as shown in Examples 8—10, the sheet pack adheres strongly to dirt of the skin, old keratin of the skin, etc. The sheet is kept for about 5—20 minutes, then peeled off. As the result, the effect that these metabolism inhibitors of the skin are removed is exhibited.

Further, as shown in Examples 8—10, the adhesion is increased with time. By this, the skin is tensioned and incited. It is useful to the beauty effect.

As shown in the result of Fig. 4, in Examples 8—10, the lowering of the 4 times readhesion is not shown. The other hand, Comparative example 3 shows a remarkably lowering in the first readhesion, and the readhesion does not recover hereafter. In Comparative example 4, the early adhesion is low, and the increase of the adhesion by the readhesion is not also recognized.

As shown in Examples 8—10, the high readhesion exhibits a characteristic of the sheet pack that, as if a visitor suddenly comes when these sheet packs are applied to the face, the sheet is once peeled off, and reapplied after that, the cleansing effect is not decreased.

Patch test

In regard to Examples 1 and 9, Comparative examples 1 and 3, the patch test was conducted for 20 healthy adults who are 23—61 years old.

(Method of the patch test)

Each sample as described above was cut square of 25 × 25 mm, and applied to the inside of the arms of tested persons.

The test time was 1 hour and 24 hours, the skin was observed within 5 minutes after each test time, and the result was evaluated by the following criteria.

Criteria

| | |
|---|---|
| - | No erythema |
| ± | Very slight erythema |
| + | Well defined erythema |
| ++ | Moderate to severe erythema |
| +++ | Severe erythema to slight eschar formation |

The test result is shown in Tables 4 and 5.

Table 4 1 hour patch test result

| No. | Name | Sex | Age | Example 1 | Example 9 | Comparative example 1 | Comparative example 3 |
|---|---|---|---|---|---|---|---|
| 1 | A.A. | female | 25 | - | - | - | - |
| 2 | H.H. | " | 35 | - | - | ± | - |
| 3 | H.O. | male | 26 | - | - | - | - |
| 4 | M.I. | " | 26 | - | - | - | - |
| 5 | S.O. | " | 44 | - | - | - | - |
| 6 | M.K. | " | 23 | - | - | - | - |
| 7 | H.K. | " | 34 | - | - | - | - |
| 8 | T.K. | " | 24 | - | - | - | - |
| 9 | Y.K. | " | 41 | - | - | - | - |
| 10 | M.S. | " | 23 | - | - | - | - |
| 11 | I.T. | " | 50 | - | - | - | - |
| 12 | H.D. | " | 49 | - | - | - | - |
| 13 | M.T. | " | 25 | - | - | - | - |
| 14 | K.N. | " | 40 | - | - | - | - |
| 15 | S.M. | " | 42 | - | - | - | - |
| 16 | T.M. | " | 38 | - | - | - | - |
| 17 | S.M. | " | 33 | - | - | - | - |
| 18 | S.Y. | " | 42 | - | - | - | - |
| 19 | T.Y. | " | 61 | - | - | - | - |
| 20 | T.Y. | " | 41 | - | - | - | - |

Table 5 24 hours patch test result

| No. | Name | Sex | Age | Example 1 | Example 9 | Comparative example 1 | Comparative example 3 |
|---|---|---|---|---|---|---|---|
| 1 | A.A. | female | 25 | - | - | - | - |
| 2 | M.I. | male | 26 | - | - | - | - |
| 3 | H.O. | " | 26 | - | - | - | - |
| 4 | S.O. | " | 44 | - | - | - | - |
| 5 | H.O. | " | 49 | - | - | - | - |
| 6 | M.K. | " | 23 | - | - | - | - |
| 7 | H.K. | " | 34 | - | - | - | - |
| 8 | T.K. | " | 24 | - | - | - | - |
| 9 | Y.K. | " | 41 | - | - | - | - |
| 10 | M.S. | " | 23 | - | - | + | + |
| 11 | K.T. | " | 34 | - | - | - | - |
| 12 | H.D. | " | 49 | - | - | ± | - |
| 13 | M.T. | " | 25 | - | - | - | - |
| 14 | Y.N. | " | 47 | - | - | - | - |
| 15 | N.F. | " | 45 | - | - | - | - |
| 16 | S.M. | " | 42 | - | - | - | - |
| 17 | T.M. | " | 38 | - | - | - | - |
| 18 | S.M. | " | 33 | - | - | - | - |
| 19 | S.Y. | " | 42 | - | - | - | - |
| 20 | T.Y. | " | 41 | - | - | - | - |

From these tables, in Comparative example 1, 1 hour patch test shows that 1 case per 20 cases produces slightly erythema, and 24 hours patch test shows that 2 cases per 20 cases produces erythema. In Comparative example 3, 24 hours patch test shows that 2 cases to one case per 20 cases produce erythema.

On the contrary, Examples 1 and 9, both 1 hour patch test and 24 hours patch test show that no case produces erythema. From the results, it is recognized that the cataplasma and the pack agents of this invention give little stimulate for the skin.

## Claims

1. A hydrated adhesive gel using a product which is obtained by reacting an aqueous solution essentially containing protein selected from gelatin, proteose, pepton, casein, albumin, globulin, prolamin, protamine, histone and glutelin, together with a N-hydroxyimidoester compound represented by the following formula (1).

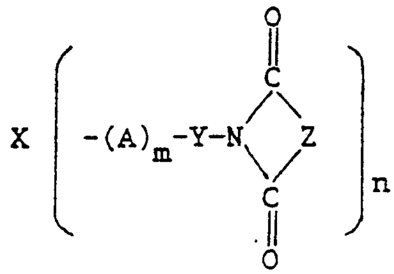

.... (1)

wherein X is a residue of a compound having 2 to 6 carbons and 2 to 6 hydroxyl groups, A is one or more groups selected from oxyethylene, oxypropylene, and oxybutylene groups,

Y is a residue of dibasic acid selected from oxalic acid, malonic acid, succinic acid, glutaric acid, adipic acid, azelaic acid, sebacic acid, decane-dicarboxylic acid, iso succinic acid, methyl succinic acid, malic acid, maleic acid, fumaric acid, tartaric acid, acetondicarboxylic acid, itaconic acid, phthalic acid, isophthalic acid, terephthalic acid, homophthalic acid, hexahydrophthalic acid, tetrahydrophthalic acid, dihydrophthalic acid, p-phenylenediacetic acid, naphthalene-2,3-dicarboxylic acid, diphenic acid, aspartic acid or glutamic acid.

Z is

① a group of (2)

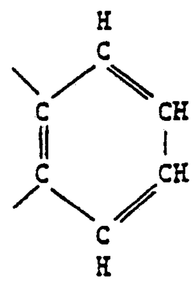

and including compounds of this structure which have been partially substituted

② a group of (3)

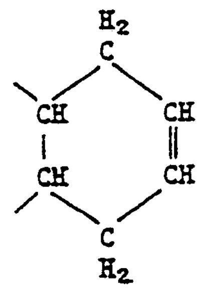

and including compounds of this structure which have been partially substituted

③ a group of (4)

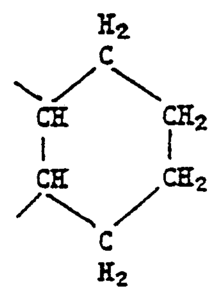

and including compounds of this structure which have been partially substituted

④ a group of (5)

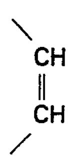

and including compounds of this structure which have been partially substituted

⑤ a group of (6)

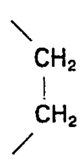

and including compounds of this structure which have been partially substituted

⑥ a group of (7) 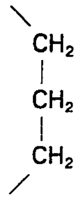 and including compounds of this structure which have been partially substituted

⑦ a group of (8) 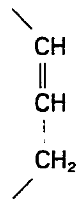 and including compounds of this structure which have been partially substituted

m is 1—3000

n is 2—6

2. A hydrated adhesive gel using a product which is obtained by reacting an aqueous solution essentially containing protein selected from gelatin, proteose, pepton, casein, albumin, globulin, prolamin, protamine, histone and glutelin, a gelling retarder, and a hydrophilic tackifier together with a N-hydroxyimidoester compound represented by the following formula (1).

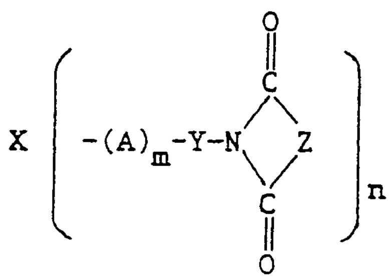 .... (1)

wherein X is a residue of a compound having 2 to 6 carbons and 2 to 6 hydroxyl groups, A is one or more groups selected from oxyethylene, oxypropylene, and oxybutylene groups,

Y is a residue of dibasic acid selected from oxalic acid, malonic acid, succinic acid, glutaric acid, adipic acid, azelaic acid, sebacic acid, decane-dicarboxylic acid, iso succinic acid, methyl succinic acid, malic acid, maleic acid, fumaric acid, tartaric acid, acetondicarboxylic acid, itaconic acid, phthalic acid, isophthalic acid, terephthalic acid, homophthalic acid, hexahydrophthalic acid, tetrahydrophthalic acid, dihydrophthalic acid, p-phenylenediacetic acid, naphthalene-2,3-dicarboxylic acid, diphenic acid, aspartic acid or glutamic acid.

Z is

① a group of (2) 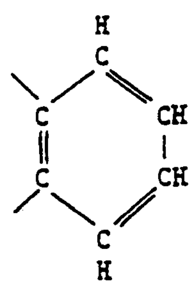 and including compounds of this structure which have been partially substituted

② a group of (3) 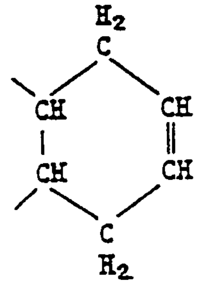 and including compounds of this structure which have been partially substituted

③ a group of (4) 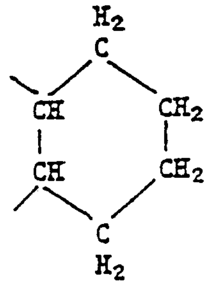 and including compounds of this structure which have been partially substituted

④ a group of (5) 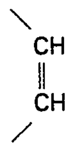 and including compounds of this structure which have been partially substituted

⑤ a group of (6) 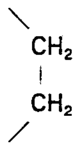 and including compounds of this structure which have been partially substituted

⑥ a group of (7) 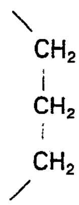 and including compounds of this structure which have been partially substituted

⑦ a group of (8) 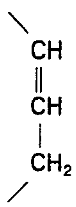 and including compounds of this structure which have been partially substituted

m is 1—3000

n is 2—6

3. A hydrated adhesive gel as defined in claim 2, wherein the gelling retarder is a compound selected from the group of both an inorganic compound which is selected from chlorine, bromine, nitrate group, and an organic compound which is selected from resorcinol, hydroquinone, pyrochatecol, pyrogallol, alcohols, urea and furfural.

4. A hydrated adhesive gel as defined in claim 2, wherein the hydrophilic tackifier is selected from the group consisting of glycerol, ethylene glycol, propylene glycol, polyethylene glycol and polypropylene glycol which are liquid a ordinary temperatures.

5. A hydrated adhesive gel as defined in claim 1, wherein a hydrated adhesive gel is selected from the group consisting of ointment and plaster.

**Patentansprüche**

1. Hydriertes Haftgel, das eine Verbindung enthält, die bei der Reaktion einen wässrigen Lösung, die im wesentlichen Protein aus Gelatine, Protease, Pepton, Casein, Albumin, Globulin, Prolamin, Protamin, Histon und Glutelin enthält, mit N-hydroxyimidoester entsteht und durch folgende Formel (1) wiedergeben wird:

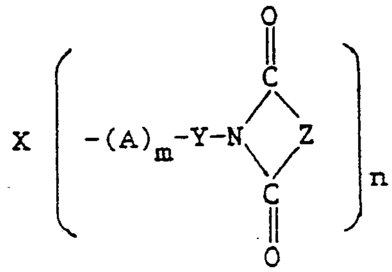

.... (1)

wobei X einen Rest einer Verbindung von 2 bis 6 Kohlenstoffatomen und 2 bis 6 Hydroxylgruppen darstellt, A ist eine oder mehrere aus Oxyethylen, Oxypropylen und Oxybutylen ausgewählte Gruppen,

Y ist ein Rest einer zweiwertigen Säure, ausgewählt aus Oxalsäure, Malonsäure, Bernsteinsäure, Glutarsäure, Adipinsäure, Azelainsäure, Sebacinsäure, Dekan-Dicarboxylsäure, Isobernsteinsäure, Methylbernsteinsäure, Apfelsäure, Maleinsäure, Fumarsäure, Weinsteinsäure, Acetondicarboxylsäure, Itakonsäure, Phthalsäure, Isophthalsäure, Terephthalsäure, Homophthalsäure, Hexahydrophthalsäure, Tetrahydrophthalsäure, Dihydrophthalsäure, p-Phenylendiessigsäure, Naphthalin-2,3-dicarboxylsäure, 2,2'-Diphenyldicarbonsäure, Asparginsäure oder Glutaminsäure,

Z ist

① eine Gruppe von (2)

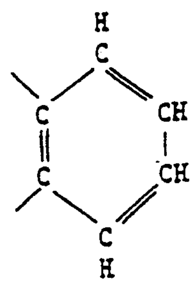

und schließt Verbindungen dieser Struktur ein, bei denen teilweise substituiert wurde,

② eine Gruppe von (3)

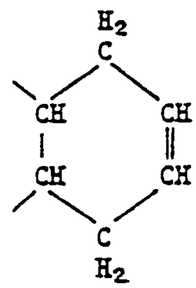

und schließt Verbindungen dieser Struktur ein, bei denen teilweise substituiert wurde,

③ eine Gruppe von (4)

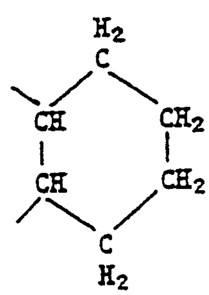

und schließt Verbindungen dieser Struktur ein, bei denen teilweise substituiert wurde,

④ eine Gruppe von (5)

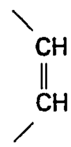

und schließt Verbindungen dieser Struktur ein, bei denen teilweise substituiert wurde,

⑤ eine Gruppe von (6)

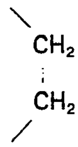

und schließt verbindungen dieser Struktur ein, bei denen teilweise substituiert wurde,

⑥ eine Gruppe von (7)

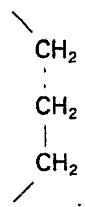

und schließt Verbindungen dieser Struktur ein, bei denen teilweise substituiert wurde,

⑦ eine Gruppe von (8)

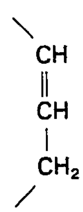

und schließt Verbindungen dieser Struktur ein, bei denen teilweise substituiert wurde,

wobei m 1—3000 und
n 2—6 ist.

2. Hydriertes Haftgelt, das eine Verbindung enthält, die bei der Reaktion einer wässrigen Lösung, die im wesentlichen Protein aus Gelatine, Protease, Pepton, Casein, Albumin, Globulin, Prolamin, Protamin, Histon und Glutelin einen Gelierungsverzögerer und einen hydrophilen Haftvermittler enthält, mit N-hydroxyimidoester entsteht und durch folgende Formel (1) wiedergegeben wird

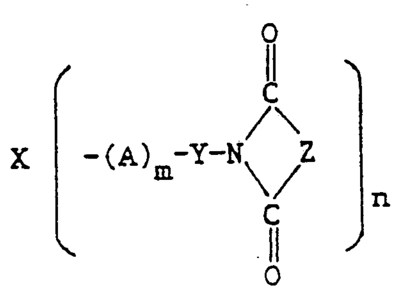

.... (1)

wobei X einen Rest einer Verbindung von 2 bis 6 Kohlenstoffatomen und 2 bis 6 Hydroxylgruppen darstellt, A ist eine oder mehrere aus Oxyethylen, Oxypropylen und Oxybutylen ausgewählte Gruppen,

Y ist ein Rest einer zweiwertigen Säure, ausgewählt aus Oxalsäure, Malonsäure, Bernsteinsäure, Glutarsäure, Adipinsäure, Azelainsäure, Sebacinsäure, Dekan-Dicarboxylsäure, Isobernsteinsäure,

Methylbernsteinsäure, Apfelsäure, Maleinsäure, Fumarsäure, Weinsteinsäure, Acetondicarboxylsäure, Itakonsäure, Phthalsäure, Isophthalsäure, Terephthalsäure, Homophthalsäure, Hexahydrophthalsäure, Tetrahydrophthalsäure, Dihydrophthalsäure, p-Phenylendiessigsäure, Naphthalin-2,3-dicarboxylsäure, 2,2'-Diphenyldicarbonsäure, Asparginsäure oder Glutaminsäure,

Z ist

① eine Gruppe von (2)

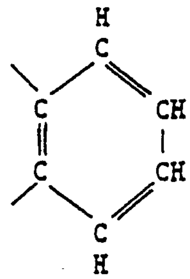

und schließt Verbindungen dieser Struktur ein, bei denen teilweise substituiert wurde,

② eine Gruppe von (3)

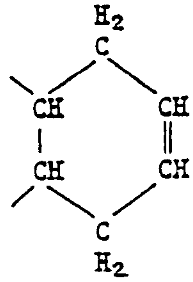

und schließt Verbindungen dieser Struktur ein, bei denen teilweise substituiert wurde,

③ eine Gruppe von (4)

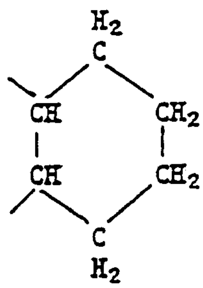

und schließt Verbindungen dieser Struktur ein, bei denen teilweise substituiert wurde,

④ eine Gruppe von (5)

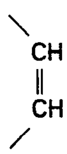

und schließt Verbindungen dieser Struktur ein, bei denen teilweise substituiert wurde,

⑤ eine Gruppe von (6)

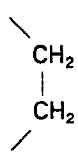

und schließt verbindungen dieser Struktur ein, bei denen teilweise substituiert wurde,

⑥ eine Gruppe von (7)

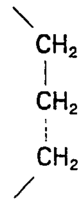

und schließt Verbindungen dieser Struktur ein, bei denen teilweise substituiert wurde,

⑦ eine Gruppe von (8)

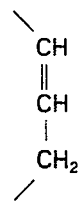

und schließt Verbindungen dieser Struktur ein, bei denen teilweise substituiert wurde,

wobei m 1—3000 und
n 2—6 ist.

3. Hydriertes Haftgel nach Anspruch 2, dadurch gekennzeichnet, daß der Gelierungsverzögerer eine Verbindung aus einer anorganischen Komponente, ausgewählt aus der Gruppe der Chloride, Bromide und Nitrate sowie einer organischen Komponente, ausgewählt aus Resorcin, Hydrochinon, Pyrochatecol, Pyrogallol, Alkoholen, Urea und Furfural ist.

4. Hydriertes Haftgel nach Anspruch 2, dadurch gekennzeichnet, daß der hydrophile Haftvermittler aus einer Gruppe bestehend aus Glycerin, Ethylenglykol, Propylenglykol, Polyethylenglykol und Polypropylenglykol, die bei Raumtemperatur flüssig vorliegen, ausgewählt ist.

5. Hydriertes Haftgel nach Anspruch 1, dadurch gekennzeichnet, daß ein hydriertes Haftgel ausgewählt ist, welches als Salben und Pflaster angewendet wird.

**Revendications**

1. Gel adhésif hydraté utilisant un produit qui est obtenu en faisant réagir une solution aqueuse contenant essentiellement une protéine choisie parmi la gélatine, la protéose, la peptone, la caséine, l'albumine, la globuline, la prolamine, la protamine, l'histone et la glutéline avec un N-hydroxyimidoester représenté par la formule (1) suivante:

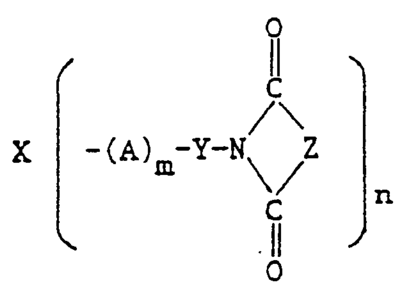

.... (1)

dans laquelle X est un radical d'un composé en $C_2$ à $C_6$ ayant 2 à 6 groupes hydroxyle, A est un ou plusieurs groupes choisis parmi les groupes oxyéthylène, oxypropylène et oxybutylène,

Y est un radical d'un diacide choisi parmi l'acide oxalique, l'acide malonique, l'acide succinique, l'acide glutarique, l'acide adipique, l'acide azélaïque, l'acide sébacique, l'acide décane-dicarboxylique, l'acide isosuccinique, l'acide méthylsuccinique, l'acide malique, l'acide maléique, l'acide fumarique, l'acide tartrique, l'acide acétonedicarboxylique, l'acide itaconique, l'acide phtalique, l'acide isophtalique, l'acide téréphtalique, l'acide homophtalique, l'acide hexahydrophtalique, l'acide tétrahydrophtalique, l'acide dihydrophtalique, l'acide p-phénylènediacétique, l'acide naphtalène-2,3-dicarboxylique, l'acide diphénique, l'acide aspartique, et l'acide glumatique.

① un groupe (2)

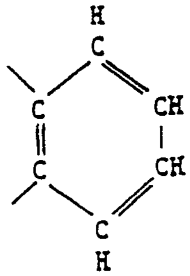

y compris des composés présentant cette structure qui ont été partiellement substitués.

② un groupe (3)

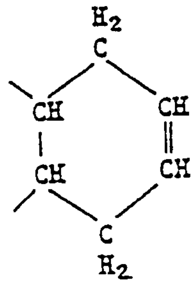

y compris des composés présentant cette structure qui ont été partiellement substitués.

③ un groupe (4)

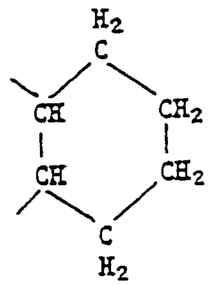

y compris des composés présentant cette structure qui ont éte partiellement substitués.

④ un groupe (5)

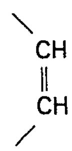

y compris des composés présentant cette structure qui ont été partiellement substitués.

⑤ un groupe (6)

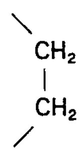

y compris des composés présentant cette structure qui ont été partiellement substitués.

⑥ un groupe (7)

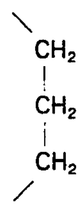

y compris des composés présentant cette structure qui ont été partiellement substitués.

⑦ un groupe (8)

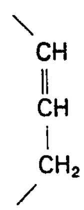

y compris des composés présentant cette structure qui ont été partiellement substitués.

m est 1—3000

n est 2—6

2. Gel adhésif hydraté utilisant un produit qui est obtenu en faisant réagir une solution aqueuse

contenant essentiellement une protéine choisie parmi la gélatine, la protéose, la peptone, la caséine, l'albumine, la globuline, la prolamine, la protamine, l'histone et la glutéline, un retardateur de gélification et un agent d'adhésivité hydrophyle avec un un N-hydroxyimidoester représenté par la formule (1) suivante:

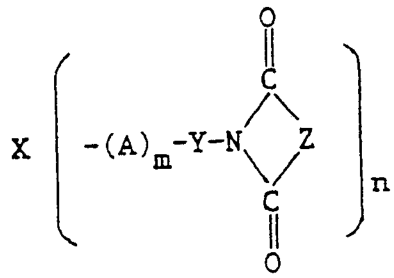

.... (1)

dans laquelle X est un radical d'un composé en $C_2$ à $C_6$ ayant 2 à 6 groupes hydroxyle, A est un ou plusieurs groupes choisis parmi les groupes oxyéthylène, oxypropylène et oxybutylène,

Y est un radical d'un diacide choisi parmi l'acide oxalique, l'acide malonique, l'acide succinique, l'acide glutarique, l'acide adipique, l'acide azélaïque, l'acide sébacique, l'acide décane-dicarboxylique, l'acide isosuccinique, l'acide méthylsuccinique, l'acide malique, l'acide maléique, l'acide fumarique, l'acide tartrique, l'acide acétonedicarboxylique, l'acide itaconique, l'acide phtalique, l'acide isophtalique, l'acide téréphtalique, l'acide homophtalique, l'acide hexahydrophtalique, l'acide tétrahydrophtalique, l'acide dihydrophtalique, l'acide p-phénylènediacétique, l'acide naphtalène-2,3-dicarboxylique, l'acide diphénique, l'acide aspartique, et l'acide glumatique.

Z est

① un groupe (2)

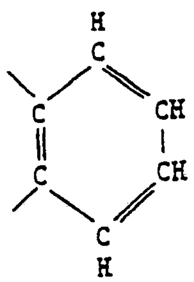

y compris des composés présentant cette structure qui ont été partiellement substitués.

② un groupe (3)

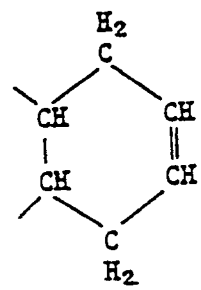

y compris des composés présentant cette structure qui ont été partiellement substitués.

③ un groupe (4)

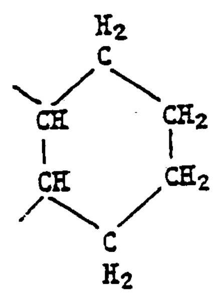

y compris des composés présentant cette structure qui ont éte partiellement substitués.

④ un groupe (5)

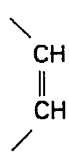

y compris des composés présentant cette structure qui ont été partiellement substitués.

⑤ un groupe (6) 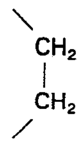 y compris des composés présentant cette structure qui ont été partiellement substitués.

⑥ un groupe (7) 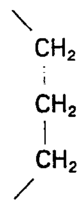 y compris des composés présentant cette structure qui ont été partiellement substitués.

⑦ un groupe (8) 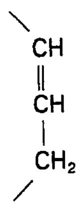 y compris des composés présentant cette structure qui ont été partiellement substitués

m est 1—3000

n est 2—6

3. Gel adhésif hydraté suivant la revendication 2, dans lequel le retardateur de gélification est un composé choisi dans le groupe constitué à la fois d'un composé minéral qui est choisi parmi le chlore, le brome, un groupe nitrate et d'un composé organique qui est choisi parmi le résorcinol, l'hydroquinone, le pyrochatecol, le pyrogallol, des alcools, l'urée et le furfural.

4. Gel adhésif hydraté suivant la revendication 2, dans lequel l'agent d'adhésivité hydrophile est choisi parmi le glycérol, l'éthylene glycol, le propylène glycol, le polyéthylène glycol et le polypropylène glycol qui sont liquides à la température ambiante.

5. Gel adhésif hydraté tel que défini dans la revendication 1, dans lequel le gel hydraté est choisi parmi une pommade et du plâtre.

FIG_1

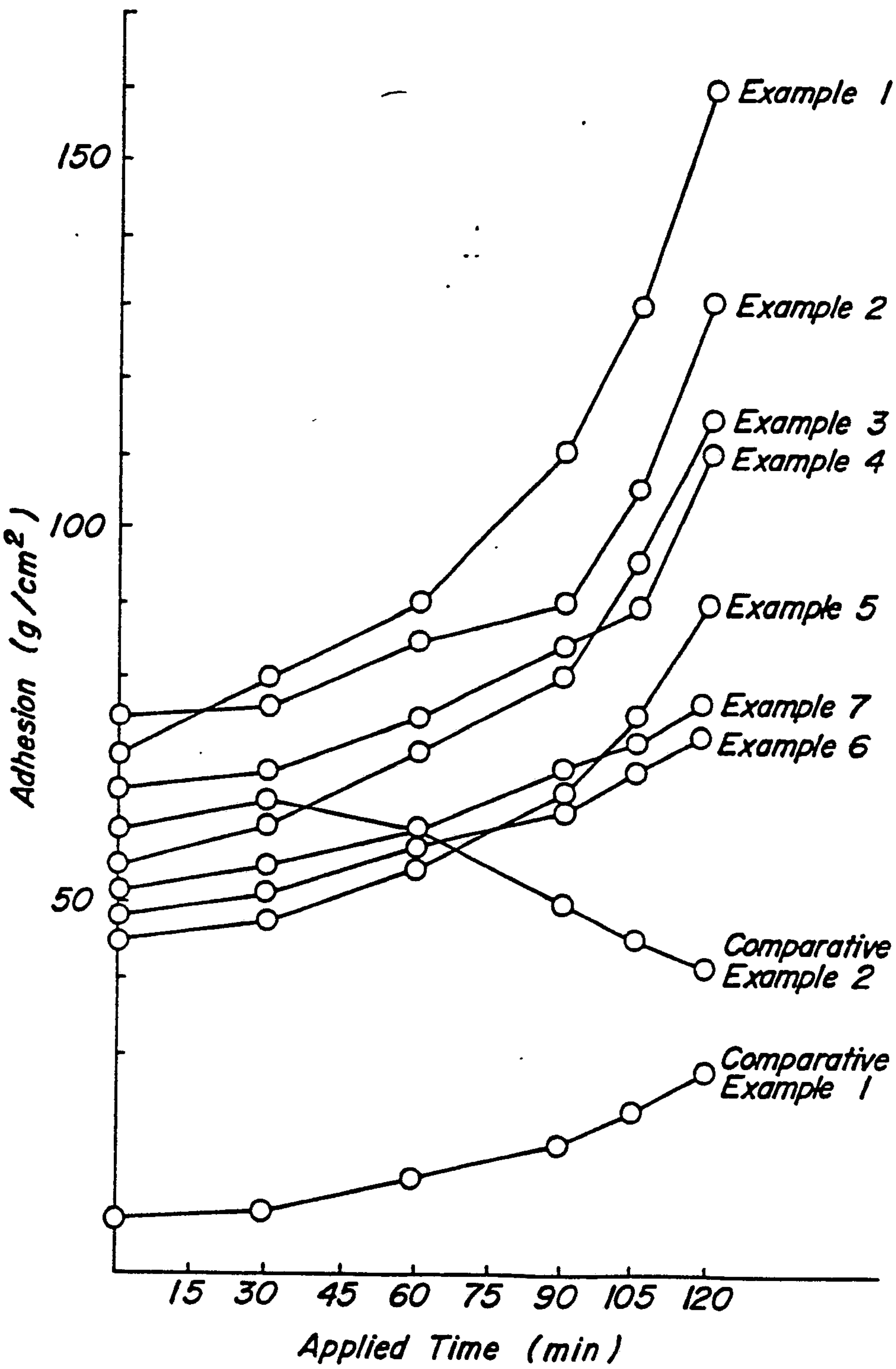
Example 1
Example 2
Example 3
Example 4
Example 5
Example 7
Example 6
Comparative Example 2
Comparative Example 1
150
100
50
Adhesion (g/cm²)
15 30 45 60 75 90 105 120
Applied Time (min)

FIG_2

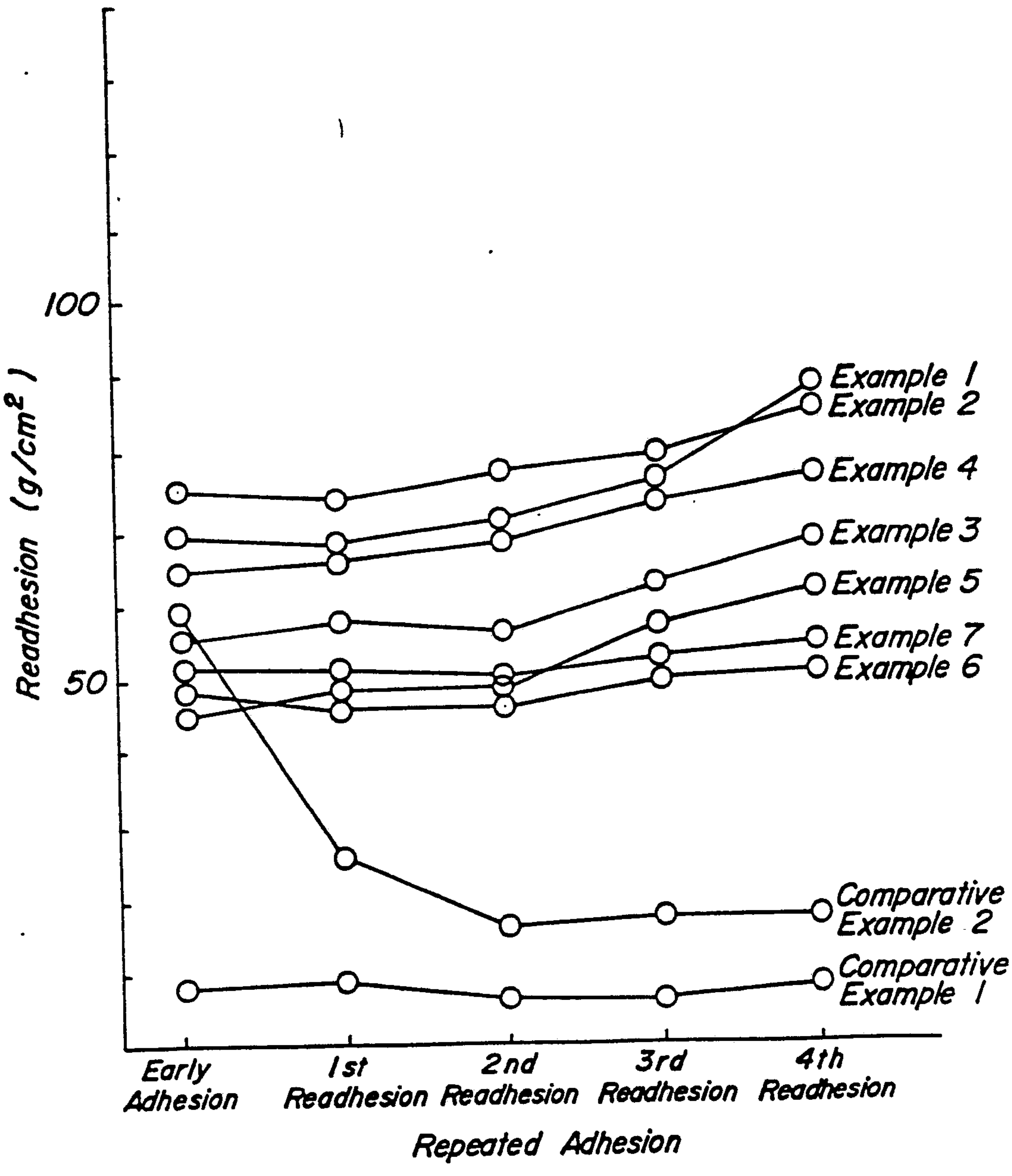
100
50
Readhesion (g/cm²)
Example 1
Example 2
Example 4
Example 3
Example 5
Example 7
Example 6
Comparative Example 2
Comparative Example 1
Early Adhesion
1st Readhesion
2nd Readhesion
3rd Readhesion
4th Readhesion
Repeated Adhesion

FIG.3

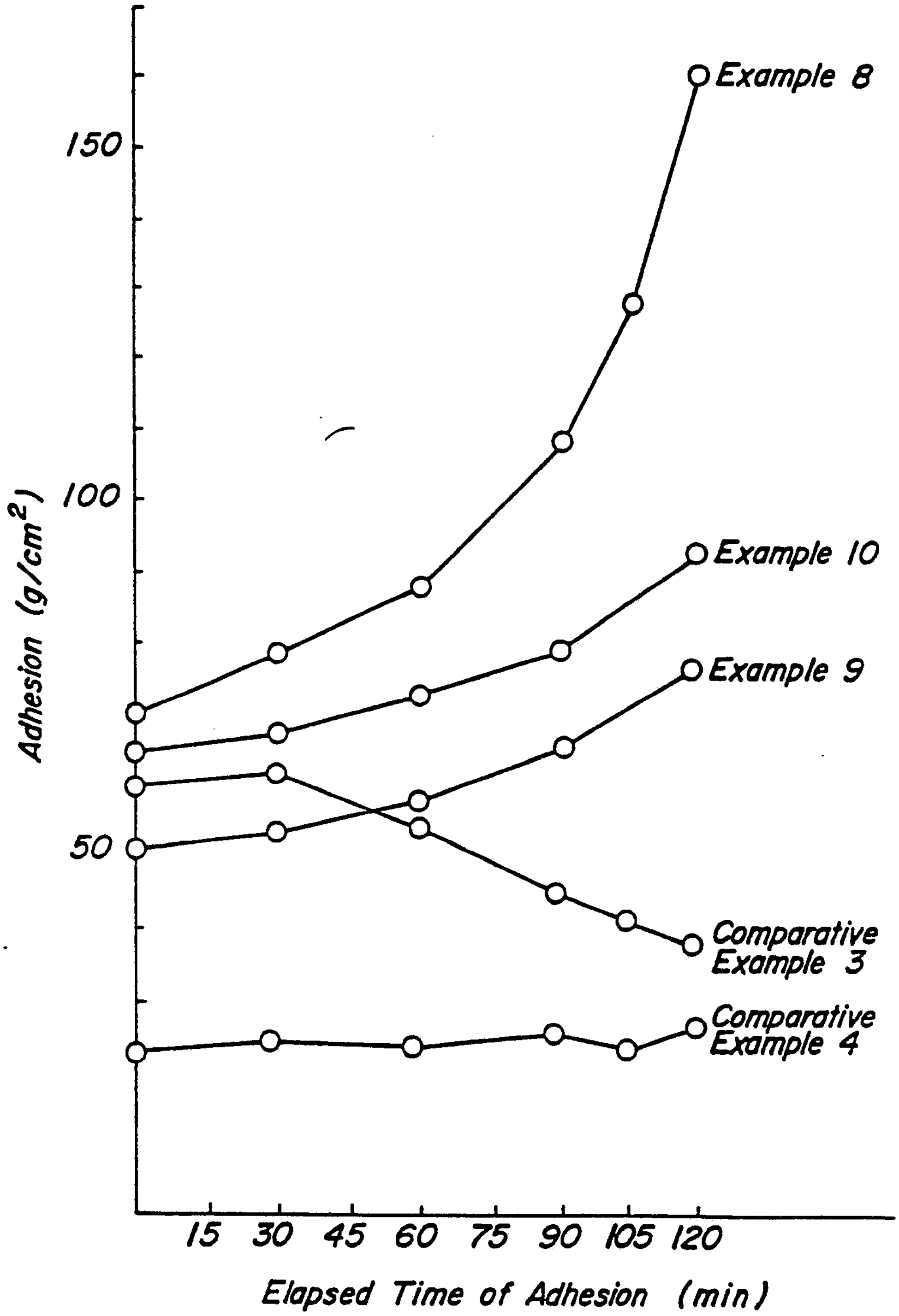
Example 8
Example 10
Example 9
Comparative Example 3
Comparative Example 4
150
100
50
Adhesion (g/cm2)
15
30
45
60
75
90
105
120
Elapsed Time of Adhesion (min)

FIG. 4

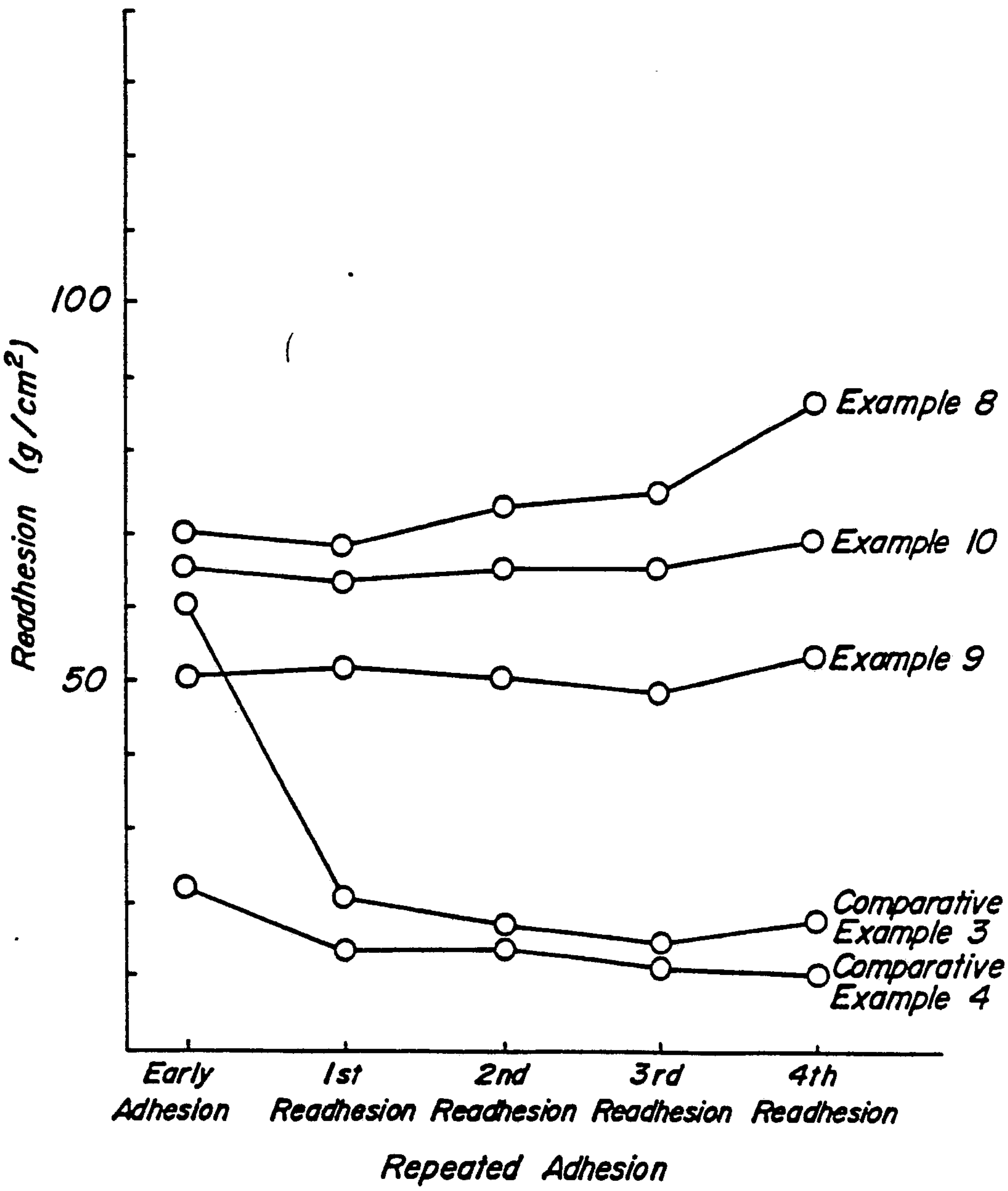
100
50
Readhesion (g/cm²)
Example 8
Example 10
Example 9
Comparative Example 3
Comparative Example 4
Early Adhesion
1st Readhesion
2nd Readhesion
3rd Readhesion
4th Readhesion
Repeated Adhesion